# EUROPEAN PATENT APPLICATION

(11) **EP 4 831 300 A1**
(43) Date of publication of application: **28.10.2026**
(21) Application number: 25172328.4
(22) Date of filing: 24.04.2025
(51) Int. Cl.: C12Q 1/6883

(54) **ATYPICAL DUSP GENES AND OBSTRUCTIVE SLEEP APNEA**

(71) Applicant: Universiteit Antwerpen, 2000 Antwerpen (BE); Universitair Ziekenhuis Antwerpen, 2650 Edegem (BE)
(72) Inventor: VAN EYCK, Annelies, 2000 Antwerpen (BE); VERHULST, Stijn, 2000 Antwerpen (BE); OGUNJIMI, Benson, 2650 Edegem (BE); MEYSMAN, Pieter, 2000 Antwerpen (BE); AFFATICATI, Fabio, 2000 Antwerpen (BE); VAN HOORENBEECK, Kim, 2650 Edegem (BE)
(74) Representative: Arnold & Siedsma

(57) **Abstract**

The present invention is situated in the field of diagnosing obstructive sleep apnea (OSA). Provided herein is a method for diagnosing and/or predicting the risk of OSA by determining expression of atypical DUSP genes in a biological test sample from a subject at risk or suspected having OSA. Further provided is a combination of atypical DUSP genes for use in the diagnosis and/or prediction of the risk of OSA as well as a diagnostic kit for detecting the expression of said atypical DUSP genes.

## Description

### FIELD OF THE INVENTION

The present invention is situated in the field of diagnosing obstructive sleep apnea (OSA). Provided herein is a method for diagnosing and/or predicting the risk of OSA by determining expression of atypical DUSP genes in a biological test sample from a subject at risk or suspected having OSA. Further provided is a combination of atypical DUSP genes for use in the diagnosis and/or prediction of the risk of OSA as well as a diagnostic kit for detecting the expression of said atypical DUSP genes.

### BACKGROUND TO THE INVENTION

Obstructive sleep apnea (OSA) is characterized by repeated episodes of breathing pauses (apneas and hypopneas) during sleep caused by upper airway obstruction resulting in intermittent hypoxia (IH), increased arousals from sleep and sleep fragmentation. In the general pediatric population, the prevalence of OSA is estimated around 2-3%, however OSA can be present in 13 to 60% of children with obesity. Similar to obesity, OSA is independently associated with important morbidity, mostly involving the cardiovascular and metabolic systems. Although efforts have been made for developing efficient approaches to understand the pathophysiological mechanisms of OSA, the current diagnosis of OSA remains poor.

The disadvantage of existing methods for diagnosing OSA are complex, uncomfortable and time-consuming. The gold standard for diagnosing OSA is polysomnography (PSG), or, sleep study. This test is performed while the patient is asleep at a sleep laboratory, and monitors brain waves, blood oxygen levels, heart rate and breathing, as well as eye and leg movements by applying sensors to the body. However, the sleep test itself does not provide the location of the obstruction, so in some cases invasive evaluation methods of the upper airway are warranted to identify potential sites of collapse that lead to OSA. For example, nasopharyngoscopy is a procedure in which a flexible fiberoptic endoscope is introduced through the nose and throat to observe anatomical structures that narrow the airway and compromise airflow and cause snoring. Sleep endoscopy is similar to nasopharyngoscopy, however it is performed under mild sedation (with a hypnotic drug, such as propofol) and it is an outpatient procedure.

In particular for pediatric subjects such as toddlers and young children, an overnight sleep test, applying adhesive sensors to the body, and using invasive techniques is generally experienced as unsuitable and undesirable by parents and the child. Thus, there is an urgent need for a non-invasive, fast and reliable method to diagnose OSA, in particular in pediatric subjects. It was therefore an object to better understand the pathogenetic mechanisms involved in OSA to provide an improved and accurate non-invasive or minimally invasive molecular test method for the diagnosis of OSA. As a means thereto, the inventors performed whole blood mRNA sequencing on biological samples of sleep apnea patients and healthy controls and identified differentially expressed DUSP genes.

Dual-specificity phosphatases (DUSPs) possess the biological activity of dephosphorylating both threonine/serine and tyrosine residues of their substrates and come in two flavors: typical and atypical DUSPs. Typical DUSPs (e.g. DUSP1, DUSP2) are also known as MAPK-specific phosphatases (MKPs) and structurally contain (i) the MAP kinase-binding (MKB) domain in the N-terminal end which in its turn comprises the kinase-interacting motif (KIM) that interacts with the common docking domain of MAPKs to mediate the enzyme-substrate interaction; and (ii) the conserved DUSP catalytic domain in the C-terminal end. Atypical DUSPs are identified by their structural similarity with that of typical DUSPs (i.e. they contain the conserved DUSP catalytic domain in the C-terminal end) but they lack the N-terminal MKB domain with the KIM motif. Therefore, atypical DUSPs are usually smaller in size compared to typical DUSP proteins. Further, atypical DUSPs can function on other phosphorylated protein substrates and can dephosphorylate nonprotein substrates such as RNA or lipids.

The inventors have unexpectedly found that one or more atypical DUSP genes have excellent diagnosing and/or predictive performance making these genes suitable for use in a method for diagnosing and/or predicting OSA, in particular in pediatric subjects. In another aspect of the invention, the targeting of DUSP genes or proteins may contribute to the development of therapeutic treatments for alleviating the symptoms of people with OSA.

### SUMMARY OF THE INVENTION

This document is based, in part, on the discovery of molecules (e.g., nucleic acids) that are differentially expressed or regulated between sleep apnea patients and healthy controls. This document also is based, in part, on the discovery that the levels of nucleic acid expression can be used to distinguish subjects with sleep apnea from healthy subjects. The analysis of atypical DUSP genes for OSA diagnosis offers new insights into OSA pathology and improved diagnostic accuracy. In particular, the expression levels of genes listed in **Table 2** can be assessed to determine a sleep apnea signature and diagnose obstructive sleep apnea.

In a first aspect, the present invention provides a method of diagnosing and/or predicting the risk of obstructive sleep apnea (OSA), said method comprising:
a) providing a biological test sample from a subject at risk or suspected having OSA; and
b) determining the expressing of one or more atypical DUSP genes; and
c) wherein the expression of said atypical DUSP genes is compared to a reference value and wherein an alteration in expression in said biological test sample compared to said reference value is indicative for OSA.

In a further embodiment of the method of the invention, the subject is a subject with overweight or obesity or a pediatric subject, but in a specific embodiment the subject is an pediatric subject with overweight or a pediatric subject with obesity.

In a particular embodiment of the method of the invention, the atypical DUSP genes encode for a gene product having a molecular weight of less than 30 kDa, in particular less than 27 kDa, more in particular less than 25 kDa.

In a very specific embodiment of the method of the invention, expression is determined of at least atypical DUSP21. In still a further embodiment, the method comprises further determining the expression of at least one more atypical DUSP genes or at least one or more typical DUSP genes, or a combination of one or more atypical DUSP genes and one or more typical DUSP genes.

In a specific embodiment, the method comprises further determining the expression of one or more genes selected from the list comprising: DUSP3, DUSP11, DUSP12, DUSP13, DUSP15, DUSP18, DUSP19, DUSP22, DUSP23, DUSP24, DUSP27, DUSP28, DUSP14, DUSP26, DUSP1, DUSP2, DUSP4, DUSP5, DUSP6, DUSP7, DUSP8, DUSP9, DUSP10, DUSP16, XIST, MAP7D2, POLR3D, SLC43A3, KLRC3, DAAM2, USP9Y, KDM5D, TTTY15, BCORP1, in particular XIST, POLR3D, MAP7D2, or any combination thereof.

In a particular embodiment of the present method, said alteration in expression is a decrease in expression of one or more atypical DUSP genes as compared to a reference value and is indicative for OSA. In a specific embodiment, the reference value is the level of expression of the one or more atypical DUSP genes from a subject who does not have OSA. In some embodiments of the method of the invention, the expression of one or more atypical DUSP genes is normalized to one or more normalization genes or using count normalization.

In a further embodiment of the method of the invention, expression is determined of RNA transcripts, in particular mRNA transcripts. Preferably biological test samples may be used selected from the list comprising of: (whole) blood, serum, plasma, saliva, or urine.

Another aspect of the invention relates to a combination of DUSP21 and one or more genes selected from the list comprising: DUSP3, DUSP11, DUSP12, DUSP13, DUSP15, DUSP18, DUSP19, DUSP22, DUSP23, DUSP24, DUSP27, DUSP28, DUSP14, DUSP26, DUSP1, DUSP2, DUSP4, DUSP5, DUSP6, DUSP7, DUSP8, DUSP9, DUSP10, DUSP16, XIST, MAP7D2, POLR3D, SLC43A3, KLRC3, DAAM2, USP9Y, KDM5D, TTTY15, BCORP1, in particular XIST, POLR3D and/or MAP7D2, for use in the diagnosis and/or prediction of the risk of OSA.

In a further aspect, provided herein is a diagnostic kit for performing the method according to the invention wherein the kit comprises agents for detecting the expression of at least one or more atypical DUSP genes defined herein.

In a still another aspect of the invention, a method for the reduction and/or treatment of OSA is provided comprising the step of administering to a subject in need thereof an effective amount of a composition comprising one or more atypical DUSP polypeptides or small molecules, or a nucleic acid construct encoding for one or more atypical DUSP polypeptides or small molecules.

In a particular embodiment, the at least one DUSP polypeptides or small molecule is DUSP21.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.
**Figure 1****. Volcano plot differential expression results for the categorical case.** To capture genes with a relevant effect size, log 2 Fold Change thresholds were set at an absolute value of 0.5 while the adjusted p-value threshold was set at 0.05.
**Figure 2****. Mann-Whitney testing confirms a difference in the median expression of DUSP21 between the positive and negative sleep apnea conditions.** The significant differences between the genes associated with the four largest log 2 fold changes, are reported here. P-values of the relative Mann-Whitney tests are also shown.

**Figure 3****: Volcano plot differential expression results for the continuous case.** To capture genes with a relevant effect size, log 2 Fold Change thresholds were set at an absolute value of 0.5 while the adjusted p-value threshold was set at 0.05.
**Figure 4****: Spearman's rank correlation.** Testing does not highlight a strong and significant relationship between DUSP21 and the average number of sleep apnea events.
**Figure 5****: Library size plotting.** Graph reveals probable low-quality samples due to shallow sequencing.
**Figure 6****: Principal Component Analysis (PCA).** Graph shows a sizable separation in the transcriptomics data revealed to be a batch effect caused by a technical artifact. The x and y axis represent respectively the first and second PC. The first component, PC1, captures 25.25% of the explained variance. The scheme indicates the experimental sequencing dates. Sample IDs are annotated on the figure. The batch effect is rather large so that the number of differentially expressed genes, if we were to conduct the analysis between these two groups, would be 10705.
**Figure 7****: Mann-Whitney testing.** Relationship between gender and the number of nightly hypoxic events. Significance was reached with a p-value of 0.001.
**Figure 8****: Spearman correlation analysis.** Analysis between the apnea-hypopnea index and several of the most commonly considered risk-factors of sleep apnea. Correlation coefficients and relative p-value are also shown. The risk-factors in order: (A) Age, (B) Waist circumference, (C) Glucose level, (D) Insulin level and (E) BMI.
**Figure 9****: Mann-Whitney testing.** Library sizes between the two observed batch groups reveals a significant disparity likely cause by technical differences. Significance was reached with a p-value of 0.0005.
**Figure 10****: Baseline log2 Counts per Million (CPM) density plot.** After low-read-transcripts removal confirm outlier behavior for some samples.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As used in the specification and the appended claims, the singular forms "a", "an", and "the" include plural referents unless the context clearly dictates otherwise. By way of example, "a compound" means one compound or more than one compound.

The terms "comprising", "comprises" and "comprised of" as used herein are synonymous with "including", "includes" or "containing", "contains", and are inclusive or open-ended and do not exclude additional, non-recited members, elements, or method steps. The terms also encompass "consisting of" and "consisting essentially of".

The term "about" or "approximately" as used herein when referring to a measurable value such as a parameter, an amount, a temporal duration, and the like, is meant to encompass variations of +/-10% or less, preferably +/-5% or less, more preferably +/- 1 % or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

The terms described above and others used in the specification are well understood to those in the art. All references, and teachings specifically referred to, cited in the present specification are hereby incorporated by reference in their entirety.

### General

As already detailed herein above, the overall objective of the present invention is to provide a more accurate sleep apnea signature and a more convenient, non-invasive and more sensitive method for diagnosing and/or predicting the risk of obstructive sleep apnea (OSA). The present invention addresses this and other needs by providing an improved and accurate non-invasive or minimally invasive molecular test method for the diagnosis of OSA based on quantification of atypical DUSP gene expression in biological test samples obtained from a subject at risk or suspected of having such a disorder. In addition, the use of the methods disclosed herein can also allow the development of new effective therapeutic and/or preventive treatments for OSA.

Accordingly, in a first aspect, the present invention provides a method of diagnosing and/or predicting the risk of obstructive sleep apnea (OSA), said method comprising:
a) providing a biological test sample from a subject at risk or suspected having OSA; and
b) determining the expressing of one or more atypical DUSP genes; and
c) wherein the expression of said atypical DUSP genes is compared to a reference value and wherein an alteration in expression in said biological test sample compared to said reference value is indicative for OSA.

### Obstructive sleep apnea

As used herein and unless otherwise specified, the term **"obstructive sleep apnea"** (OSA) or "obstructive sleep apnea syndrome" are used interchangeably and refer to a sleep-related breathing disorder characterized by recurrent episodes of complete or partial obstruction of the upper airway. The obstruction in the airway is caused excessive relaxation of the throat muscles, causing the airway to narrow or close. This leads to reduces oxygen levels in the blood and fragmented sleep as the brain tends to wake the person to resume normal breathing. Common symptoms include, but are not limited to, respiratory pauses, noisy breathing sounds, paradoxical breathing movements, cyanosis, restless sleep, excessive diaphoresis, sleep enuresis, (loud) snoring, gasping or choking during sleep, excessive daytime sleepiness, morning headaches, difficulty concentrating, and irritability. Risk factors for developing OSA include overweight, obesity, a large neck circumference, a narrowed airway, being male, older age, and a family history of the condition.

OSA differs from a less severe condition known as habitual or primary snoring (PS), which is typically considered to be a more benign form of upper airway resistance. PS occurs in the absence of gas exchange abnormalities or evidence of snore-associated alterations in sleep architecture. Currently, the ability to accurately diagnose OSA and distinguish between OSA and PS requires an overnight sleep study, or polysomnography, which is not readily available to many subjects due to the cost and the availability of a suitable sleep diagnostic facility. Thus, a subject may be afflicted with OSA for an extended period of time without an accurate diagnosis and subsequent treatment, which can thus cause the adverse consequences associated with OSA to be long-lasting or permanent.

As such, in one aspect, the presently-disclosed subject matter provides methods and gene expression signatures that can be utilized to diagnose OSA, and distinguish it from for example PS, without the need of an overnight sleep study, thereby providing earlier treatment opportunities. In some embodiments, the presently-disclosed subject matter includes methods and kits for diagnosing OSA in a subject, and for determining whether to initiate or continue prophylaxis or treatment of OSA in a subject, by identifying at least one atypical DUSP gene associated with OSA in a biological sample from the subject.

### Atypical DUSP genes

Dual-specificity phosphatases (DUSPs) possess the biological activity of dephosphorylating both threonine/serine and tyrosine residues of their substrates and come in two flavors: typical and atypical DUSPs. As used herein, **"typical DUSP genes"** are to be understood as MAPK-specific phosphatases (MKPs) that structurally contain a MAP kinase-binding (MKB) domain in the N-terminal end and a conserved DUSP catalytic domain in the C-terminal end. The MKB domain comprises the kinase-interacting motif (KIM) that interacts with the common docking domain of MAPKs to mediate the enzyme-substrate interaction.

As used herein and unless otherwise specified, the term **"atypical DUSP genes"** refers to a subset of the Dual-specificity Phosphatase gene family, which encode enzymes that dephosphorylate both serine/threonine and tyrosine residues on their target proteins. Atypical DUSP genes play a crucial role in the regulation of various cell processes, including cell growth, differentiation, and stress responses such as oxidative stress response, inflammatory response, heat shock response, and DNA damage response. Atypical DUSPs differ from typical DUSPs in their structure as well as their function as atypical DUSP genes may be regulated differently possibly through distinct signaling pathways or cellular contexts. More in particular, atypical DUSPs lack certain domains that are present in typical DUSPs, such as the Mitogen-Activated Protein Kinase (MAPK) binding domain, which affects their substrate specificity and regulatory mechanisms. The roles of atypical DUSP genes can be more context-dependent and may involve regulation of other signaling pathways, contributing to processes like immune responses, cell cycle control, and apoptosis.

Accordingly, where a typical DUSP contains a MAP kinase-binding (MKB) domain comprising a KIM motif, the atypical DUSP gene lacks such terminal MKB domain with the KIM motif.

Examples of members of the **typical DUSP** gene family comprises of genes from the following list: DUSP1/MKP1 (or DUSP1), DUSP2, DUSP4/MKP2 (or DUSP4), DUSP5, DUSP6/MKP3 (or DUSP6), DUSP7, DUSP8, DUSP9/MKP4 (or DUSP9), DUSP10/MKP5 (or DUSP10) and DUSP16/MKP7 (or DUSP16).

Examples of members of the **atypical DUSP** gene family comprises of genes from the following list: DUSP3, DUSP11, DUSP12, DUSP13, DUSP14/MKP6 (or DUSP14), DUSP15, DUSP18, DUSP19, DUSP21, DUSP22, DUSP23, DUSP24/STYXL1 (or DUSP24), DUSP26/MKP8 (or DUSP26), DUSP27, and DUSP28. DUSP11, DUSP15, DUSP24 show moderate expression; and DUSP23 shows high expression.

In another embodiment of the method of the present invention, expression levels are determined of one or more atypical DUSP genes that encode for a gene product having a molecular weight of less than 30 kDa, such as less than 29 kDa, 28 kDa, 27 kDa, 26 kDa, in particular less than 25 kDa, such as less than 24 kDa, 23 kDa, 22 kDa, 21 kDa, more in particular less than 23 kDa.

In another embodiment of the method of the present invention, expression levels are determined from one or more atypical DUSP genes that encode for a gene product having a molecular weight of about and between 19 kDa and 30kDa, in particular about and between 20 kDa and 26 kDa, more in particular about and between 21 kDa and 25 kDa.

In a specific embodiment of the method of the present invention, the expression is determined of at least atypical DUSP21. It has been found by the inventors that determining expression of at least atypical DUSP21 gene provides excellent diagnostic performance (AUC of more than 0.8) for discriminating subjects having, or subject suspected to have, OSA from normal or healthy subjects. In particular, the DUSP21 gene was the strongest discriminator and was downregulated in individuals experiencing sleep apnea events with a -7.8 Log2FoldChange and adjusted p-value = 0.0002 (as exemplified in Example 1). Receiver operating characteristic (ROC) analysis was used to determine the diagnostic performance of the individual or combinations of DUSP genes as obtained by the area under the curve (AUC) for distinguishing subjects with a diagnosis of a OSA from subjects who do not have OSA.

In a specific embodiment, the expression of atypical DUSP21 is determined further in combination with the expression of one or more genes selected from the list comprising: DUSP3, DUSP11, DUSP12, DUSP13, DUSP15, DUSP18, DUSP19, DUSP22, DUSP23, DUSP24, DUSP27, DUSP28, DUSP14, DUSP26, DUSP1, DUSP2, DUSP4, DUSP5, DUSP6, DUSP7, DUSP8, DUSP9, DUSP10, DUSP16, XIST, MAP7D2, POLR3D, SLC43A3, KLRC3, DAAM2, USP9Y, KDM5D, TTTY15, BCORP1, or any combination thereof, in particular DUSP1.

In a more specific embodiment, the expression of atypical DUSP21 is determined further in combination with the expression of one or more atypical DUSP genes from the following list comprising: DUSP3, DUSP11, DUSP12, DUSP13, DUSP14/MKP6, DUSP15, DUSP18, DUSP19, DUSP22, DUSP23, DUSP24/STYXL1, DUSP26/MKP8, DUSP27, and DUSP28, in particular DUSP3, DUSP11, DUSP12, DUSP13, DUSP15, DUSP18, DUSP19, DUSP22, DUSP23, and DUSP28 or any combination thereof.

In a very specific embodiment, a method for diagnosing and/or predicting the risk of a OSA is provided by determining at least the expression of one or more of the gene combinations selected from the group comprising of: DUSP21/DUSP23;, providing excellent diagnostic performance as reflected with an AUC of more than 0.75.

Particularly useful DUSP genes used in the context of the invention are DUSP1, DUSP2, DUSP3, DUSP5, DUSP6, DUSP10, DUSP11, DUSP12, DUSP14, DUSP16, DUSP18, DUSP19, DUSP21, DUSP22, DUSP23, and/or DUSP28 and are represented by nucleic acid sequences SEQ ID NO: 1-35 or having at least 85% sequence variation thereto; or by amino acid sequences SEQ ID NO: 36-124, or having at least 85% sequence variation thereto. In some embodiments, the expression of atypical DUSP21 is detected having a nucleic acid sequence as set forth in SEQ ID NO: 34 or 35, or having at least 85% sequence variation thereto; or an amino acid sequence as set forth in SEQ ID NO: 124 or having at least 85% sequence variation thereto. In some instances assembly GRCh38.p14 or assembly T2T-CHM13v2.0 is used.

### Other combinations

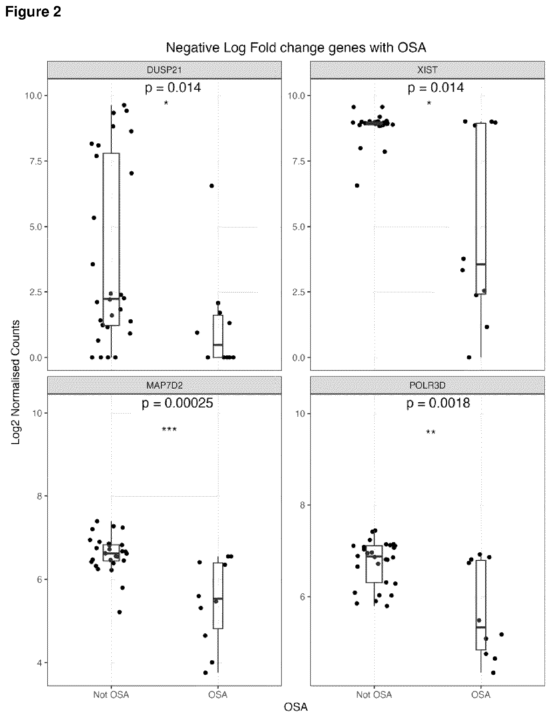

In even a further embodiment, it might be advantageous to determine the expression of any one of the following genes comprising: XIST, MAP7D2, POLR3D, SLC43A3, KLRC3, DAAM2, USP9Y, KDM5D, TTTY15, BCORP1, in particular XIST, POLR3D and/or MAP7D2. It has further been found by the inventors that determining expression of at least atypical DUSP21 gene in combination with either XIST, POLR3D or MAP7D2, or any combination thereof provides excellent diagnostic performance for discriminating subjects having, or subject suspected to have, OSA from normal or healthy subjects (Figure 2).

Accordingly, in one aspect of the invention, the present invention provides a method for diagnosing and/or predicting the risk of OSA, said method comprising:
a) providing a biological test sample from a subject at risk or suspected having OSA; and
b) determining the expression of at least one or more of the gene combinations selected from the group comprising of: DUSP21/XIST; DUSP21/MAP7D2; DUSP21/POLR3D; DUSP21/XIST/MAP7D2; DUSP21/XIST/POLR3D; DUSP21/ MAP7D2/ POLR3D;
c) optionally further determining the expression of one or more further atypical DUSP genes selected from the list comprising: DUSP3, DUSP11, DUSP12, DUSP13, DUSP14, DUSP15, DUSP18, DUSP19, DUSP22, DUSP23, DUSP24, DUSP26, DUSP27, and DUSP28, or any combination thereof;
d) wherein the expression of said target genes is compared to a reference value and wherein an alteration in expression in said biological test sample compared to said reference value is indicative for OSA.

**"XIST"** refers to the *X-inactive specific transcript* gene, which plays a crucial role in the process of X-chromosome inactivation in female mammals. **"MAP7D2"** refers to the *Microtubule-Associated Protein 7 Domain Containing* 2 gene, which encodes a protein involved in the stabilization and organization of microtubules, which are components of the cell's cytoskeleton. **"POLR3D"** refers to *DNA-directed RNA polymerase III subunit RPC4* gene and complements a temperature-sensitive mutant isolated from the BHK-21 Syrian hamster cell line. It leads to a block in progression through the G1 phase of the cell cycle at nonpermissive temperatures.

In another embodiment, the method according to the invention may further include performing Polysomnography (PSG) on the subject having or suspected of having OSA. In yet another embodiment, the method can further include determining Apnea Hypopnea Index for the subject having or suspected of having OSA.

### Subjects

In the context of the present application, the term **"patient"** is generally synonymous with the term **"subject"** and includes all mammals at risk or suspected having OSA. Preferably, the patient is a human subject. The human subject can be an overweight subject, more in particular an obese subject.

The terms **"overweight"** and **"obese"** refer to the condition in which the subject has an abnormal or excessive amount of body fat accumulation, which is possible to affect the health of the subject in a negative way. As used herein and unless otherwise specified, a subject is generally considered as overweight when the body mass index (BMI) is over 25 and a subject is considered obese when it has a BMI of 30 or higher. In some embodiments, the subject is a pediatric subject wherein overweight or obesity is defined according to the International Obesity Task Force (IOTF) as BMI ≥ those age-sex specific BMI cut offs corresponding to resp. BMI=25 kg/m2, BMI=30 kg/m2 at age 18.

The term **"BMI"** refers to Body Mass Index, a numerical value derived from the individual's weight and height. Throughout the specification, and in case no unit is given, BMI is defined by kg/m² (weight in kilograms of said individual divided by the square of the height of said individual in meters. BMI can be calculated by (body mass/height²) and translated to BMI z-score according to M Roelants, R Hauspie, K Hoppenbrouwers, References for growth and pubertal development from birth to 21 years in Flanders (Belgium), Annals of Human Biology, 36 (6):680-694, 2009. It should be noted that BMI values or BMI z-scores are a general indicators of body fatness and does not directly measure body fat. As such, it has limitations for example when it is applied to individuals with short stature, or high muscle mass. An alternative to BMI is the waist-to-hip ratio (WHR) or the waist-to-height ratio (WHtR) which better takes into account the distribution of fat in the body. The WHR measures the ratio between the circumference of the waist and the hips wherein a higher WHR can indicate a greater amount of visceral fat.

Evidently, it is understood by the person skilled in the art that several indicators exist that can be used to determine an overweight or obese condition in a subject and that the method according to the invention is not limited to any extend to such a determination. In addition, when reference is made to specific BMI values or BMI z-scores, one will appreciate that these can be translated to a corresponding WHR or WHtR value.

In a specific embodiment, the subject has a BMI of at least 25 kg/m², such as at least 26 kg/m², at least 27 kg/m², at least 28 kg/m², at least 29 kg/m², at least 30 kg/m², at least 31 kg/m², at least 32 kg/m², at least 33 kg/m², at least 34 kg/m², at least 35 kg/m², at least 36 kg/m², at least 37 kg/m², at least 38 kg/m², at least 39 kg/m², at least 40 kg/m². In a specific embodiment, the subject is an overweight subject having a BMI between at least 25 kg/m² and 30 kg/m², or an obese subject, preferably an obese subject having a BMI between at least about 30 kg/m² and about 50 kg/m², even more preferably between about 31 kg/m² and about40 kg/m², most preferably between about 32 kg/m² and about 34 kg/m².

In another particular embodiment of the method of the invention, the subject can be a pediatric subject, an adolescent subject, and an adult subject.

The term **"pediatric subject"** refers to individuals generally under the age of 18, and includes infants, toddlers, young children and adolescents. In specific embodiment, the subject is a pediatric subject, in particular a pediatric subject having the age between about 0 to about 18 years, more in particular between about 4 to about 16 years, even more in particular between about 6 to about 15 years, most particular between about 8 to about 14 years.

In a more specific embodiment, the pedriatic subject has an age of less than 18 years, such as less than 17, 16, 15, 14, 13, 12, 11, 10, 9, 8, 7, 6, 5, 4, 3, 2 or less than 1 years.

In a very specific embodiment, the subject is an overweight pediatric subject or an obese pediatric subject. In a very specific embodiment, the subject is a pediatric subject having overweight with a BMI of 30 or more as defined according to the IOTF. In another embodiment, the subject is a pediatric subject having obesity with a BMI of 35 or more as defined according to the IOTF. It has been found that in a pediatric subpopulation with obesity, in particular a subpopulation with an age of about and between 8 to about 18 years with a BMI equal to or higher than 30 as defined according to the IOTF, the determination of the expression of one or more atypical DUSP genes, and more in particular atypical DUSP21 expression, provides excellent diagnosing and/or predictive performance for OSA.

### Biological test samples

As used herein and unless otherwise specified, the term **"biological test sample"** refers to a sample that can be taken or obtained from a subject, such as humans or animals, wherein the sample can be for example a tissue sample or a bodily fluid sample or a biopsy sample. Non-limiting examples of suitable bodily fluid samples which can be used in the methods described herein can include whole blood, plasma, serum, CSF, urine, saliva. Any method can be used to obtain a sample (e.g., blood, saliva, urine) for evaluation. For example, a sample such as blood can be obtained by peripheral venipuncture and evaluated to determine if it contains one or more atypical DUSP genes as defined herein. A particularly suitable biological sample includes a peripheral blood sample, more in particular a peripheral blood sample from which RNA can be purified.

Biological test samples as described herein can be obtained at various points of time from a subject that is at risk of, or suspected having OSA. In a particular embodiment, one or more biological samples can be taken before a sleep test study, and one or more biological samples can be taken after a sleep test study. In another embodiment, one or more biological samples can be taken before OSA manifests or is diagnosed, in particular at least 1 year but in particular at least 2 years before manifestation or diagnosis, such as at least 3 years, at least 4 years, at least 5 years, before manifestation or diagnosis. Alternatively, one or more biological test samples can be taken when OSA has manifested or has been diagnosed, optionally by another method such as polysomnography. As such, the gene profile used herein is not only predictive for early onset of OSA but also useful when the OSA is already present in the subject.

In some embodiments of the present method of the invention, two or more biological test samples can be collected several months apart, e.g., 1, 3, 6, 12, or 24 months apart, preferably 3-6 months apart, wherein the separately collected samples obtained at spaced time points are indicative for the likelihood of progression of OSA.

In another embodiment, biological test samples that are particularly suitable for the method according to the invention are biological test samples obtained from subjects in a subpopulation aged about and between 0-18 having a BMI of about and between 25 to 50.

### Determining expression

In the context of the present invention, the term **"expression"** of a gene is to be understood as the process by which the information encoded in a gene is used to synthesize a functional gene product, typically a protein, through the stages of transcription and translation as known by those skilled in the art. As used herein, the term **"determining the expressing of one or more atypical DUSP genes",** is meant to be a step in the method in which the expression level of atypical DUSP genes is determined, in order to detect the presence and/or amount of expression of such atypical DUSP genes. In any embodiment disclosed herein, the expression level can be determined on the level of protein or RNA (or mRNA) expression level, by means of suitable techniques known by those skilled in the art including but not limited to: western blotting, ELISA assays, next-generation sequencing, (RT-)PCR methods.

In a particular embodiment, the method according to the invention comprises a step of determining expression of RNA transcripts of one or more atypical DUSP genes, in particular mRNA transcripts. The term **"RNA transcripts"** as used herein refers to RNA molecules that are synthesized from a DNA template during the process of transcription and can be of various types, including mRNA, rRNA, tRNA and non-coding RNAs or the alike.

In a particular embodiment, RNA or mRNA expression levels can be determined in for example bodily fluid samples by means of suitable techniques including but not limited to: polymerase chain reaction (PCR)-based detection (e.g., stem-loop reverse transcription-polymerase chain reaction [RT-PCR], quantitative RT-PCR based array method [qPCR-array]), microarray analysis, tag- based technologies (e.g., serial analysis of gene expression and next generation sequencing such as whole transcriptome shotgun sequencing or RNA-Seq), hybridization with selective probes, or mass spectrometry. One of the preferred types of techniques are RT-PCR-based techniques as such techniques allow to achieve good sensitivity and specificity.

In a particular embodiment of the method of the present invention, prior to measuring (m)RNA level, the (m)RNA is purified from the biological test sample, in particular from the bodily fluid sample. (m)RNAs can be isolated and purified from bodily fluids by various methods and commercial kits known in the art including but not limited to e.g., miRNeasy kit, Trizol extraction, concentration and purification on anion-exchangers, magnetic beads covered by RNA-binding substances, or adsorption of certain (m)RNAs on complementary oligonucleotides.

In some embodiments, the methods described herein can further comprise the step of reducing or eliminating degradation of the (m)RNA. In some embodiments, (m)RNA degradation in biological test samples and/or during (m)RNA purification is reduced or eliminated. Useful methods for reducing or eliminating miRNA degradation include, without limitation, adding RNase inhibitors, use of guanidine chloride, guanidine isothiocyanate, N-lauroylsarcosine, sodium dodecylsulphate (SDS), or a combination thereof. Reducing (m)RNA degradation in biological test samples is particularly important when sample storage and transportation is required prior to (m)RNA quantification.

### Reference value

In a specific embodiment, the expression of a selected atypical DUSP gene is compared to a reference value wherein an alteration in expression is indicative for OSA. As used herein and unless otherwise specified, the term **"reference value"** refers to a standard expression level which is used for comparison and is a typical or expected value established for a subject without OSA, or an expression level in a normal/healthy subject without OSA as determined by a medical professional and/or research professional using established methods as described herein, and/or a known expression level obtained from literature.

In a specific embodiment, said reference value is a level of expression of the one or more atypical DUSP genes from a subject who does not have OSA.

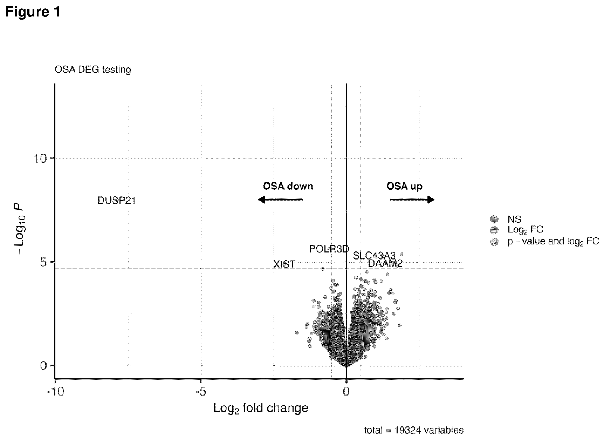

In a particular embodiment, the alteration in expression level of one or more atypical DUSP genes can be an upregulation (increase) or downregulation (decrease) compared to a reference value wherein respectively a high expression or low expression is indicative for OSA. Specific reference is made to Figure 1 wherein the atypical DUSP21 gene shows a decrease in expression pattern.

In a specific embodiment, the alteration in expression is a decrease in expression of one or more atypical DUSP genes, in particular a decrease in expression of DUSP21, as compared to a reference value and is indicative for OSA.

In another embodiment, the reference value can also refer to the expression level for a subject without obstructive sleep apnea at the time the sample is obtained from the subject, but who later exhibits obstructive sleep apnea. In this context, a change in expression within a subject from visit to visit can indicate an increased or decreased risk for OSA. For example, a plurality of expression levels can be obtained from a plurality of samples obtained from the same subject and used to identify differences between the pluralities of expression levels in each sample. Thus, in some embodiments, two or more samples obtained from the same subject can provide a gene expression signature and a reference expression level(s) of the atypical DUSP genes.

### Normalizer

In one embodiment, in case expression levels are determined on the level of (m)RNA expression level, the expression of one or more atypical DUSP genes may be normalized to one or more normalization genes or using a count normalization. Count normalization methods are particularly useful to make accurate comparisons of gene expression between samples.

In this context, the term **"normalized genes",** are genes that are consistently expressed at stable levels across different tissues, cell types, and experimental conditions but are not related to a target pathology. Particularly in gene expression studies such as quantitative PCR (qPCR), normalization genes are used to ensure that the expression levels of target genes can be accurately measured and compared. In a particular embodiment, normalizer genes that can be suitably used in the method according to the invention, are ACTB, GAPDH and/or B2M.

In another embodiment, the expression of one or more atypical DUSP genes may be normalized using a count normalization method. Non-liming normalization methods may be CPM (counts per million), TPM (transcripts per kilobase million), RPKM/FPKM (reads/fragments per kilobase of exon per million reads/fragments mapped), DESeq2's median of ratios, EdgeR's trimmed mean of M values (TMM), but in particular DESeq2 normalisation.

The DESeq2's method is a method wherein counts are divided by sample-specific size factors determined by median ratio of gene counts relative to geometric mean per gene. The accounted factors are sequencing depth and RNA composition and it is particularly suitable for gene count comparisons between samples and for DE analysis.

### Combination for use

In another aspect of the invention, a combination for use in the diagnosis and/or prediction of the risk of OSA is provided comprising DUSP21 and one or more genes selected from the list comprising: DUSP3, DUSP11, DUSP12, DUSP13, DUSP15, DUSP18, DUSP19, DUSP22, DUSP23, DUSP24, DUSP27, DUSP28, DUSP14, DUSP26, DUSP1, DUSP2, DUSP4, DUSP5, DUSP6, DUSP7, DUSP8, DUSP9, DUSP10, DUSP16, XIST, MAP7D2, POLR3D, SLC43A3, KLRC3, DAAM2, USP9Y, KDM5D, TTTY15, BCORP1, in particular XIST, POLR3D and/or MAP7D2 or any combination thereof. In a specific embodiment, a combination for use in the diagnosis and/or prediction of the risk of OSA is provided comprising any one or DUSP21/DUSP23; DUSP21/XIST; DUSP21/MAP7D2; DUSP21/POLR3D or any combination thereof.

### Diagnostic kit

In conjunction with the above diagnostic methods, the present disclosure also provides various kits comprising one or more primer and/or probe sets specific for the detection of target (m)RNA. Such kits can further include primer and/or probe sets specific for the detection of normalizer (m)RNA. A kit of the invention can also provide reagents for primer extension and amplification reactions. For example, in some embodiments, the kit may further include one or more of the following components: a reverse transcriptase enzyme, a DNA polymerase enzyme (such as, e.g., a thermostable DNA polymerase), a polymerase chain reaction buffer, a reverse transcription buffer, and deoxynucleoside triphosphates (dNTPs).

Non-limiting examples of primer or probe combinations in such kits can be as follows:
i) primer or probes specific for at least the determination of expression of atypical DUSP gene DUSP21, more in particular DUSP21/XIST; DUSP21/MAP7D2; DUSP21/POLR3D; DUSP21/XIST/MAP7D2; DUSP21/XIST/POLR3D; or DUSP21/MAP7D2/POLR3D DUSP21; or
ii) primer or probes specific for at least the determination of expression of atypical DUSP gene DUSP21, in combination with primer or probes specific for the determination of expression of one further atypical DUSP genes from the following list comprising: DUSP3, DUSP11, DUSP12, DUSP13, DUSP14, DUSP15, DUSP18, DUSP19, DUSP22, DUSP23, DUSP24, DUSP26, DUSP27, and DUSP28, or any combination thereof; or
iii) primer or probes specific for at least the determination of expression of DUSP21/XIST; DUSP21/MAP7D2; DUSP21/POLR3D; DUSP21/XIST/MAP7D2; DUSP21/XIST/POLR3D; or DUSP21/MAP7D2/POLR3D DUSP21; in combination with primer or probes specific for the determination of expression of one further atypical DUSP genes from the following list comprising: DUSP3, DUSP11, DUSP12, DUSP13, DUSP14, DUSP15, DUSP18, DUSP19, DUSP22, DUSP23, DUSP24, DUSP26, DUSP27, and DUSP28.

### Diagnosing and/or predicting

The term **"diagnosing"** as used herein means assessing whether a subject suffers from a disease as disclosed herein or not. As will be understood by those skilled in the art, such an assessment is usually not intended to be correct for all (i.e. 100%) of the subjects to be identified. The term, however, requires that a statistically significant portion of subjects can be identified.

In some embodiments, especially in cases wherein biological test samples are obtained from a subject before OSA manifests or is diagnosed, the term diagnosis may also refer to screening or predicting the onset of disease. Screening for diseases, in some embodiments, can lead to earlier diagnosis in specific cases and diagnosing the correct disease subtype can lead to adequate therapy. In another particular embodiment, the present invention provides a (m)RNA panel or OSA signature as defined herein, for use as a biomarker for diagnosis and disease surveillance.

### Treatments

The method according to the invention can further include administering a treatment for OSA to the subject diagnosed as having OSA. Suitable treatments include a lifestyle change (e.g., weight loss, regular exercise, moderate alcohol consumption, smoking reduction/cessation, a nasal decongestant, an allergy medication, change in sleeping position, avoidance of sedative medications), positive airway pressure (e.g., continuous positive airwaypressure "CPAP"; autotitrating pressure "APAP"; bilevel positive airway pressure "BPAP"), an oral appliance, and surgery.

In another aspect of the invention, a method for the reduction and/or treatment of OSA is provided, said method comprising the step of administering to a subject in need thereof an effective amount of a composition comprising one or more atypical DUSP polypeptides or small molecules, or a nucleic acid construct encoding for one or more atypical DUSP polypeptides or small molecules.

In a specific embodiment, in said method for the reduction and/or treatment of OSA, at least one DUSP polypeptides or small molecule that is administered to the subject is DUSP21.

In the context of the present application, the terms **"treatment",** "treating", "treat" and the like refer to obtaining a desired pharmacological and/or physiological effect. The effect may be prophylactic in terms of completely or partially preventing a disease or symptom thereof and/or may be therapeutic in terms of a partial or complete stabilization or cure for a disease and/or adverse effect attributable to the disease. "Treatment" covers any treatment of a disease in a mammal, in particular a human, and includes: (a) preventing the disease or symptom from occurring in a subject which may be predisposed to the disease or symptoms but has not yet been diagnosed as having it; (b) inhibiting the disease symptoms, i.e. arresting its development; or (c) relieving the disease symptom, i.e. causing regression of the disease or symptom.

As used herein, the term **"effective amount"** refers to the quantity of a substance or formulation that is sufficient to achieve the desired therapeutic or beneficial effect in a subject. This term is commonly used in the context of pharmaceuticals, nutraceuticals, and other therapeutic agents. The effective amount can vary depending on several factors, including the specific condition being treated/researched, the subject's characteristics, the route of administration, the formulation of the composition and the duration and frequency of administration.

### EXAMPLES

### EXAMPLE 1: DUSP21 expression is associated with obstructive sleep apnea in pediatric patients with obesity

### Background

Childhood obesity is one of the most important public health challenges of the 21th century, since it increases the risk of obesity at adult age1 and its associations with several comorbidities. Obesity is also a well-known risk factor for developing obstructive sleep apnea (OSA). OSA is characterized by repeated episodes of breathing pauses (apneas and hypopneas) during sleep caused by upper airway obstruction resulting in intermittent hypoxia (IH), increased arousals from sleep and sleep fragmentation. In the general pediatric population the prevalence of OSA is estimated around 2-3%, however OSA can be present in 13 to 60% of children with obesity. Similar to obesity, OSA is independently associated with important morbidity, mostly involving the cardiovascular and metabolic systems.

Obesity is characterized by a state of chronic inflammation. Increasing evidence points to a critical role of adipose tissue hypoxia in mediating the proinflammatory responses seen in obesity, which leads to systemic inflammation. In contrast, OSA is characterized by repeated cycles of hypoxia and reoxygenation, which create a characteristic pattern of nocturnal IH. This IH is seen as one of the important drivers behind the comorbidities associated with OSA as it activates the sympathetic nervous system and promotes systemic inflammation. Indeed, intermittent hypoxia in animal models exacerbates vascular dysfunction and reproduces glucose and lipid homeostasis disturbances similar as those seen in patients with OSA.

As both OSA and obesity lead to similar cardiovascular and metabolic consequences, and with increasing evidence suggesting a potential synergistic effect, it has been hypothesized that OSA shares various pathophysiological mechanisms of cardiometabolic complications with obesity. Because obesity and OSA activate similar pathways, it is difficult to determine how OSA can exacerbate the obesity-related comorbidities. Whole blood gene expression analysis could shed more light on the underlying pathophysiology of OSA, independent of obesity, and suggest possible biomarkers. Therefore, the aim of this study was to better understand the pathogenetic mechanisms involved in OSA in children with obesity through whole blood mRNA sequencing.

### Methods

### Study design

In this cross-sectional, prospective study, children and adolescents with obesity, aged 8-18 years, were consecutively enrolled through the pediatric obesity clinic of the Antwerp University Hospital between May 2017 and January 2019. Patients were excluded in case of an acute inflammatory process; use of non-steroidal anti-inflammatory drugs or immunosuppressive drugs; structural heart disease or other cardiac diseases; active malignant hematological disease; an underlying syndromic disorder; and a genetic or endogenous cause of obesity.

### Anthropometry

Height, weight, waist circumference and waist-to-hip ratio (WHR) were measured using standardized techniques by skilled personnel. Body mass index (BMI) was calculated as weight in kilograms over the square of height in m and was further analyzed as standard deviation scores (SDS) using the Flemish growth charts as a reference population. Overweight and obesity were defined according to the International Obesity Task Force criteria.

### Sleep Assessment

All children underwent nocturnal polysomnography for at least 6 hours. The following variables were continuously measured and recorded by a computerized polysomnograph (Brain RT, OSG, Rumst, Belgium): electroencephalography (C4/Al and C3/A2); electrooculography; electromyography of anterior tibial and chin muscles; and electrocardiography. Respiratory effort was measured by respiratory inductance plethysmography and oxygen saturation by a finger probe connected to a pulse oximeter (Xpod, Nonin, Minnesota, USA). Airflow was measured by means of a nasal pressure cannula and thermistor, and snoring was detected by means of a microphone at the suprasternal notch. All patients were monitored on audio/videotape using an infrared camera. Respiratory events were scored according to the American Academy of Sleep Medicine guidelines. The apnea-hypopnea index (AHI) was defined as the average number of obstructive, central and mixed apneas and hypopneas per hour of sleep. The number of obstructive apneas and hypopneas per hour of sleep were quantified in the obstructive AHI (oAHI). OSA is classified as mild (oAHI 2-5/h), moderate (>5 and up to 10/h) and severe (oAHI > 10 events/h). All desaturations of ≥3% from the baseline SaO2 were quantified and the oxygen desaturation index (ODI) was calculated as the total of desaturations divided by the total sleep time.

### Blood sample and RNA extraction

A fasting venous blood sample was drawn from all patients to determine fasting glucose, fasting insulin and high sensitivity C-reactive protein (hs-CRP) at the clinical laboratory of the Antwerp University Hospital using standardized techniques. An extra blood sample was collected in a PAXgene RNA blood tube for gene expression analysis. PAXgene samples were stored at -20°C until RNA extraction and subsequent gene expression analysis.

Total RNA was extracted from the collected whole blood samples using the PAXgene RNA blood kit following the manufacturer's instructions. To optimize RNA concentrations, the RNA clean & concentrator-5 kit was used. RNA quality was evaluated with a fragment analyzer (DNF-471 Standard Sensitivity RNA Analysis Kit) and on a Qubit Fluorometer (Qubit Broad Range RNA kit).

### 3'mRNA sequencing

All RNA samples were prepared with the QuantSeq 3' mRNA-Seq Library Prep Kit FWD for Illumina following the standard protocol for long fragments. Resulting cDNA libraries were equimolarly pooled, up to 40 samples for one NextSeq 500 sequencing run (high output v2 kit, 150 cycles, single read, Illumina).

### NGS data analysis

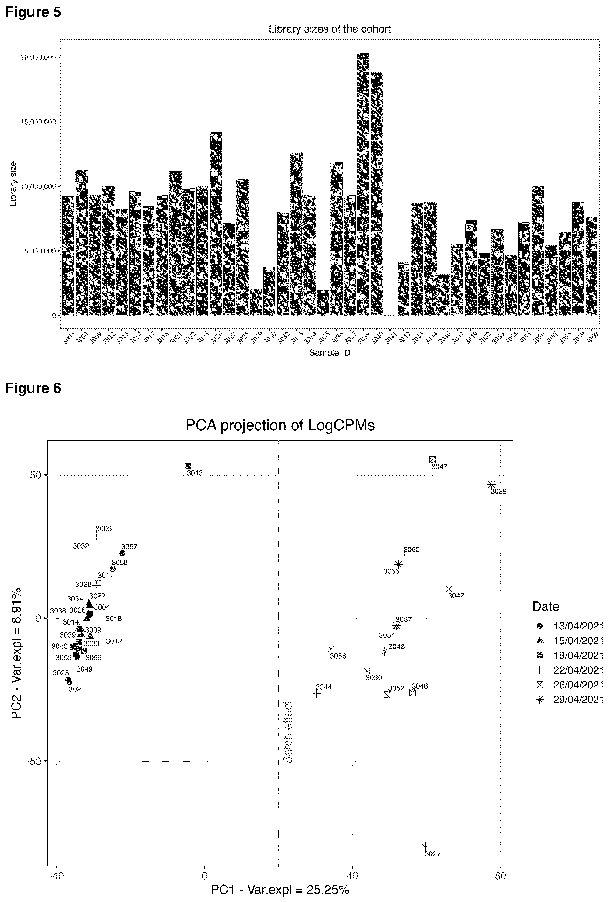

The read quality for the data was highly variable ranging from a minimum of 29540 reads for sample 3041, to a maximum of 20.45 million reads for sample 3039 **(****Figure 5****).**

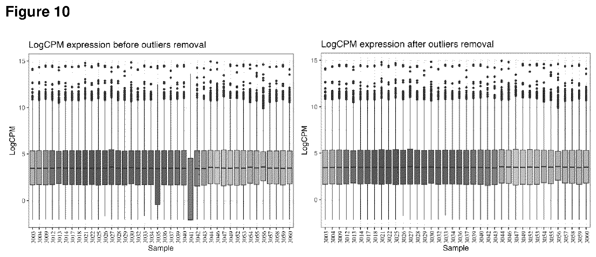

Low read transcripts were filtered out in prospect of differential expression analysis, to increase the power of the testing. Only transcripts that presented more than 1 of Log2 Counts per Million reads (the logarithmic counts that we would expect if the sample had library size of 1 million - Log2CPMs) summed across the cohort were kept. CPMs computation was performed using version 4.0.16 of edgeR. We chose to use Log2CPMs instead of raw counts to account for the different sequencing depths highlighted in **Figure 5** (and to further account for the recorded batch effect **(****Figure 10****).** From 35057 transcripts **(Figure 6A)** the number was reduced to 19331 **(Figure 6B).** Hemoglobin-related transcripts were also discarded.

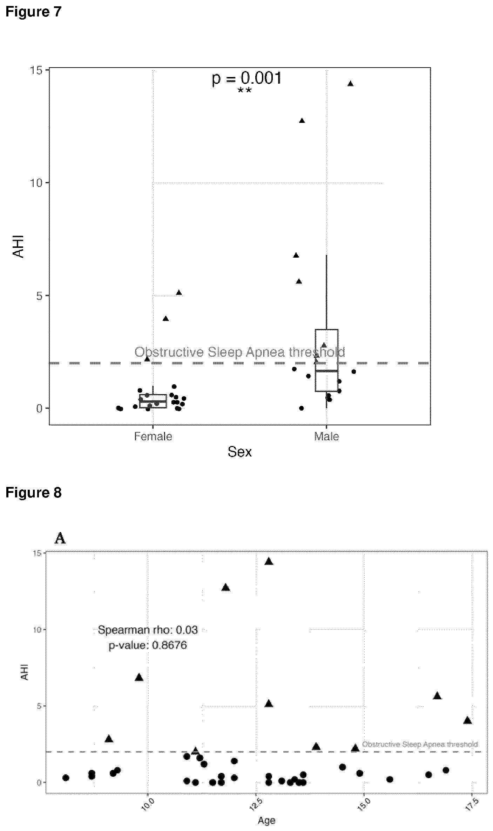

Samples that still showed an outlier behavior in terms of CPMs were therefore removed. Namely, sample 3035 and sample 3041 **(Figure 11).** After preprocessing, we performed a Principal Component Analysis (PCA) to identify possible sources of variation in the data. We thus reduced the hyperdimensional sequencing dataset to a two-dimensional space, identified by the first two PCs. This space captured the largest amount of variation in our data **(****Figure 7****).** By visualizing the sequencing date, it appeared quite evident that a technical artifact was produced by a change in the experimental setup. We thus tagged the cohort in the two emerging groups, batch one and batch two, for model correction purposes.

The differential expression analysis that followed was performed using DESeq2 (v. 1.42.1). For both cases the results were controlled by including possible confounding factors in the GLM-like formulation of DESeq2. Batches, gender, normalized age and normalized BMI were included as cofactors:$design ∼ batch + Sex + scaled Age + scaled BMI + OSA \left(or scaled oAHI\right)$

In the categorical case the effects were calculated on the binary OSA variable and in the continuous case the effects were calculated on the normalized AHI.

ClusterProfiler version 4.10.1 and the function enrichGO were used to run the Gene Ontology (GO) enrichment analysis based on the differentially expressed genes identified at the previous step, from which the most prominent ontological categories were derived. However, when studying AHI association to GO terms, only genes showing an absolute log 2 fold change of 0.5 were fed to enrichment testing and reported in **Table 1.**

Additional packages used: ggplot2 (v. 3.5.1), ggstatsplot (v. 0.12.3), ggpubr (v. 0.6.0) dplyr (v. 1.1.4) and EnhancedVolcano (v. 1.20.0). All the analyses were performed using R version 4.3.3. All data and code can be found at https://github.com/fabio-affaticati/pediatric_osa.

**Table 1: differentially expressed genes associated with the number of OSA events. Only genes showing an absolute log 2 fold change of 0.5 are reported.**

| ***Log2FoldChange*** | ***Adjusted p-value*** | ***Name*** | ***Description*** | ***Genebank*** |
|---|---|---|---|---|
| ***-2.85040*** | *0.01271* | *DUSP21* | *dual specificity phosphatase 21* | *NC_000023.11* |
| ***-1.47979*** | *0.04712* | *HIST1H2AJ* | *histone cluster 1%2C H2aj* | *NC_000006.12* |
| ***-1.04086*** | *0.00944* | *XIST* | *X inactive specific transcript (non-protein coding)* | *NC_000023.11* |
| ***-0.91085*** | *0.02892* | *RPL19P21* | *ribosomal protein L19 pseudogene 21* | *NC_000023.11* |
| ***-0.77524*** | *0.01819* | *LOC105373030* | *NoDescription* | *NC_000022.11* |
| ***-0.71861*** | *0.04095* | *GRIN38* | *glutamate receptor%2C ionotropic%2C N-methyl-D-aspartate 3B* | *NC_000019.10* |
| ***-0.71580*** | *0.01819* | *RPSAP49* | *ribosomal protein SA pseudogene 49* | *NC_000009.12* |
| ***-0.60979*** | *0.00172* | *TMPRSS6* | *transmembrane protease%2C serine 6* | *NC_000022.11* |
| ***-0.60312*** | *0.03397* | *C12orf29* | *chromosome 12 open reading frame 29* | *NC_000012.12* |
| ***-0.60099*** | *0.02109* | *FAM209A* | *family with sequence similarity 209%2C member A* | *NC_000020.11* |
| ***-0.58233*** | *0.03314* | *TPPP3* | *tubulin polymerization-promoting protein family member 3* | *NC_000016.10* |
| ***-0.55670*** | *0.03097* | *RAB4B-EGLN2* | *RAB4B-EGLN2 readthrough (NMD candidate)* | *NC_000019.10* |
| ***-0.52055*** | *0.00172* | *C4orf48* | *chromosome* 4 *open reading frame 48* | *NC_000004.12* |
| ***0.50589*** | *0.03600* | *LOC101928782* | *uncharacterized LOC101928782* | *NC_000007.14* |
| ***0.52137*** | *0.04656* | *LOC102724913* | *uncharacterized LOC102724913* | *NC_000018.10* |
| ***0.52586*** | *0.04392* | *EHF* | *ets homologous factor* | *NC_000011.10* |
| ***0.52862*** | *0.03539* | *LOC105378763* | *NoDescription* | *NC_000001.11* |
| ***0.53198*** | *0.04712* | *SCN10A* | *sodium channel%2C voltage gated%2C type X alpha subunit* | *NC_000003.12* |
| ***0.54051*** | *0.00944* | *LOC105372731* | *NoDescription* | *NC_000021.9* |
| ***0.54141*** | *0.03338* | *CXCL1* | *chemokine (C-X-C motif) ligand 1 (melanoma growth stimulating activity%2C alpha)* | *NC_000004.12* |
| ***0.54422*** | *0.02462* | *LOC102725227* | *NoDescription* | *NC_000018.10* |
| ***0.54706*** | *0.01928* | *NR4A3* | *nuclear receptor subfamily 4%2C group A%2C member* 3 | *NC_000009.12* |
| ***0.56764*** | *0.02473* | *LOC105371965* | *NoDescription* | *NC_000018.10* |
| ***0.57187*** | *0.00172* | *LOC105373470* | *NoDescription* | *NC_000001.11* |
| ***0.57379*** | *0.00524* | *FOLR3* | *folate receptor 3 (gamma)* | *NC_000011.10* |
| ***0.57784*** | *0.03314* | *LOC101927615* | *NoDescription* | *NC_000021.9* |
| ***0.57846*** | *0.00742* | *C6orf120* | *chromosome* 6 *open reading frame 120* | *NC_000006.12* |
| ***0.58031*** | *0.01379* | *AGPAT1* | *1-acylglycerol-3-phosphate O-acyltransferase 1* | *NC_000006.12* |
| ***0.58148*** | *0.02160* | *MROH5* | *maestro heat-like repeat family member 5* | *NC_000008.11* |
| ***0.58703*** | *0.00000* | *NEBL* | *nebulette* | *NC_000010.11* |
| ***0.59460*** | *0.00944* | *TINAG* | *tubulointerstitial nephritis antigen* | *NC_000006.12* |
| ***0.59623*** | *0.00944* | *LOC100507065* | *uncharacterized LOC100507065* | *NC_000012.12* |
| ***0.59736*** | *0.01242* | *LOC105376661* | *NoDescription* | *NC_000011.10* |
| ***0.60187*** | *0.01379* | *LOC101927263* | *NoDescription* | *NC_000015.10* |
| ***0.60510*** | *0.02791* | *FAM170B-AS1* | *FAM170B antisense RNA 1* | *NC_000010.11* |
| ***0.61267*** | *0.02501* | *LOC100130550* | *g2*/*M phase-specific E3 ubiquitin-protein ligase-like* | *NC_000003.12* |
| ***0.61429*** | *0.00172* | *TMEM114* | *transmembrane protein 114* | *NC_000016.10* |
| ***0.62248*** | *0.00524* | *DAAM2* | *dishevelled associated activator* of *morphogenesis 2* | *NC_000006.12* |
| ***0.62766*** | *0.01572* | T | *T%2C brachyury homolog (mouse)* | *NC_000006.12* |
| ***0.64990*** | *0.03314* | *LOC105379259* | *NoDescription* | *NC_000009.12* |
| ***0.65072*** | *0.01204* | *FLJ33534* | *uncharacterized LOC285150* | *NC_000002.12* |
| ***0.65656*** | *0.01714* | *LHFPL3-AS2* | *LHFPL3 antisense RNA 2* | *NC_000007.14* |
| ***0.66032*** | *0.01204* | *LOC105373627* | *NoDescription* | *NC_000002.12* |
| ***0.67726*** | *0.00924* | *ARG1* | *arginase 1* | *NC_000006.12* |
| ***0.68804*** | *0.00915* | *OR8A 1* | *olfactory receptor%2C family 8%2C subfamily A%2C member 1* | *NC_000011.10* |
| ***0.70125*** | *0.01204* | *LOC105371178* | *NoDescription* | *NC_000016.10* |
| ***0.79105*** | *0.00944* | *PRAMEF11* | *PRAME family member 11* | *NC_000001.11* |
| ***0.83186*** | *0.00066* | *LOC105374446* | *NoDescription* | *NC_000002.12* |
| ***0.84035*** | *0.00511* | *PACSIN3* | *protein kinase C and casein kinase substrate in neurons* 3 | *NC_000011.10* |

### Results

A total of 40 children with obesity were included with an average BMI of 31.8 ± 5.8 kg/m², which corresponds to a BMI SDS of 2.5±0.4. The mean age was 12.4 ± 2.3 years and 57.5% of the subjects were female. OSA was diagnosed in 25% of the patients. **Table** 2 shows the patient characteristic of children with and without OSA.

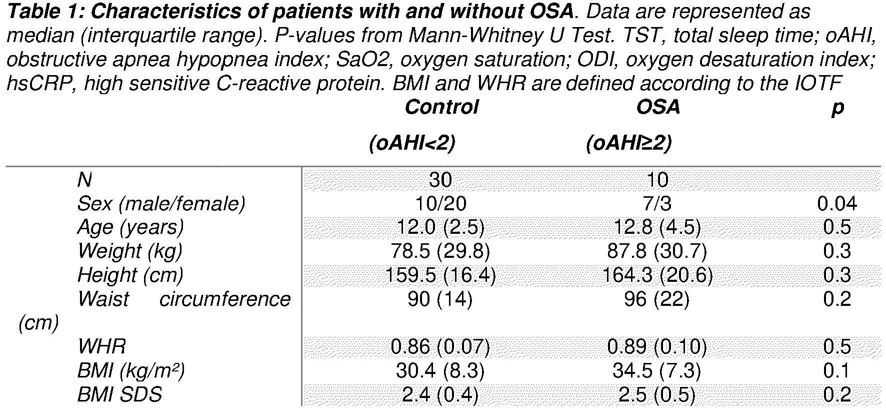

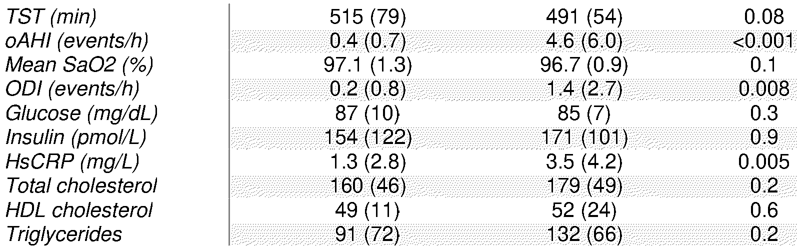

To model transcriptomic changes associated with OSA-caused hypoxia, we first segregated individuals based on the binary presence or absence of sleep apnea events (individuals considered OSA patients if oAHI >= 2). The differential expression analysis between 19331 transcripts produced 11 differentially expressed genes (DEGs), seven up-regulated and four down-regulated at the p-value adjusted cutoff for differential expression set at 0.05 **(Table 3).** In particular, the DUSP21 gene was the most strongly DEG, both in terms of absolute Log2FoldChange and adjusted p-value. DUSP21 was downregulated in individuals experiencing sleep apnea events with a -7.8 Log2FoldChange and adjusted p-value = 0.0002 following the Wald test used by DESeq2 **(****Figure 1****).**

**Table 3: differentially expressed genes positively and negatively associated with Obstructive Sleep Apnea events (n >=2 events).**

| ***Log2FoldCh ange*** | ***Adju sted p-value*** | ***Name*** | ***Description*** | ***Genebank*** |
|---|---|---|---|---|
| *-7.88380* | *0.00020* | *DUSP21* | *dual specificity phosphatase 21* | *NC_000023.11* |
| *-2.12536* | *0.03022* | *XIST* | *X inactive specific transcript (non-protein coding)* | *NC_000023.11* |
| *-0.81167* | *0.03820* | *MAP7D2* | *MAP7 domain containing 2* | *NC_000023.11* |
| *-0.59093* | *0.02058* | *POLR3D* | *polymerase (RNA) III (DNA directed) polypeptide D%2C 44kDa* | *NC_000008.11* |
| *0.95732* | *0.020587* | *SLC43A3* | *solute carrier family 43%2C member 3* | *NC_000011.10* |
| *1.24625* | *0.020587* | *KLRC3* | *killer cell lectin-like receptor subfamily C%2C member 3* | *NC_000012.12* |
| *1.33729* | *0.030227* | *DAAM2* | *dishevelled associated activator of morphogenesis 2* | *NC_000006.12* |
| *1.57112* | *0.030227* | *USP9Y* | *ubiquitin specific peptidase 9%2C Y-linked* | *NC_000024.10* |
| *1.74128* | *0.030227* | *KDM5D* | *lysine (K)-specific demethylase 5D* | *NC_000024.10* |
| *1.74862* | *0.030227* | *TTTY15* | *testis-specific transcript%2C Y-linked 15 (non-protein coding)* | *NC_000024.10* |
| *1.89955* | *0.020587* | *BCORP1* | *BCL6 corepressor pseudogene 1* | *NC_000024.10* |

The non-parametric testing of the Log 2 normalized expression of DUSP21 further corroborated the significant difference for this gene between the two conditions **(****Figure 2****).** The Mann-Whitney U reported a significant p-value of 0.014.

To better understand the relevance of the DEGs in human biology, we then analyzed the ontological categories under which DUSP21 falls, which revolve around Mitogen-Activated Protein (MAP) kinase and phosphatase activities, mainly the Gene Ontology (GO) biological process of tyrosine dephosphorylation. For the sleep apnea positively-associated categories, histone demethylation and chromatin remodeling were the most striking molecular function and biological process terms, originating from the Y chromosome encoded KDM5D gene.

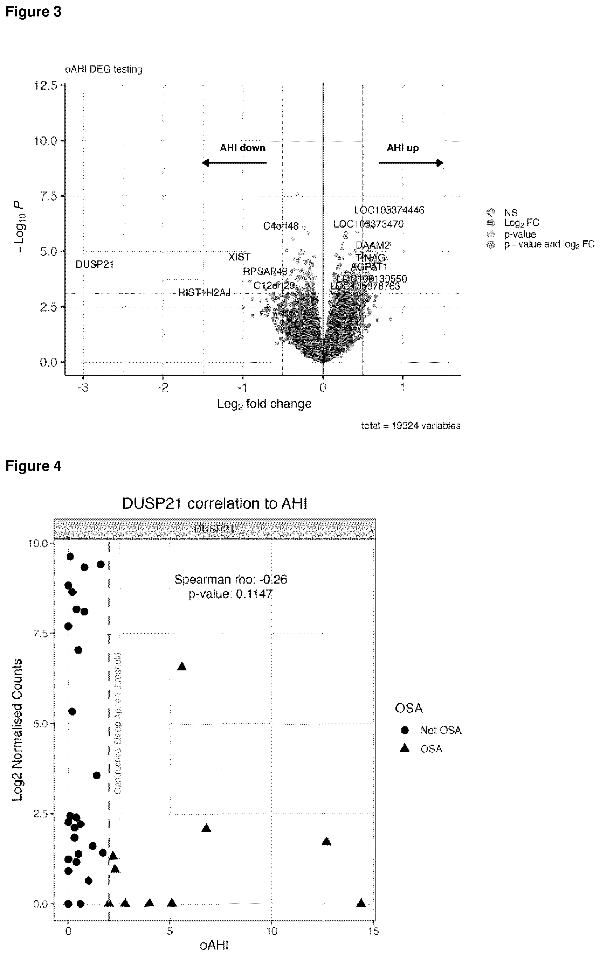

In addition, by removing the constraint of the binary membership to a sleep apnea group, we were able to discover more DEGs. We based the analysis on the continuous AHI scale and produced instead 304 DEGs with the p-value adjusted cutoff set at 0.05 **(****Figure 3****).**

The GO molecular function of MAP kinase activity is again found in the significant negative results, due to the presence of DUSP21, while the positive enrichment is dominated by granule and vesicle lumen of the Cellular Component branch of the GO.

A Spearman rank correlation test was implemented to determine the strength and directionality of the association between the normalized expression of DUSP21 and the AHI **(****Figure 4****).** However, this further testing was not significant with a p-value of 0.1 with a moderate negative association shown by a rho of -0.26.

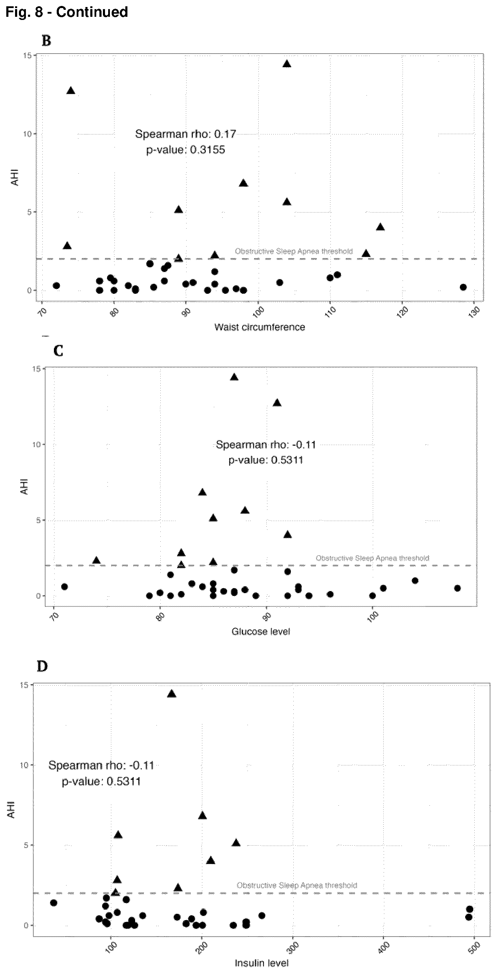

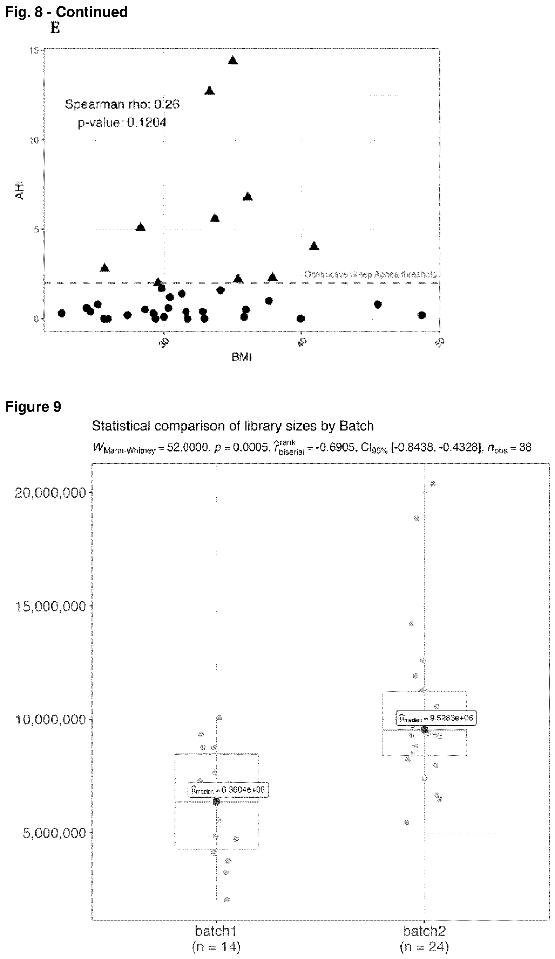

We tested for possible correlations between oAHI and demographic and clinical data of the cohort. Notably, the number of nightly hypoxic events was significantly greater for the male sub-cohort (Mann-Whitney non-parametric testing; pvalue = 0.001) **(****Figure 8****).** Correlations of AHI with other parameters that are normally considered usual associations to sleep apnea in the literature, such as BMI, Age, waist circumference, insulin and glucose levels, were not significant for our study group **Figure 9****)**

Differential expression analysis, following the same set up, was also conducted to discover associations with the Oxygen Desaturation Index (ODI - the number of times per hour of sleep that blood oxygen level dropped in the subjects below baseline) and with the oxygen saturation levels. No significant DEGs resulted from the analysis.

### Discussion

In this clinical cohort of children with obesity, we explored the pathophysiological mechanisms associated with OSA through whole blood mRNA sequencing. Here, we found 11 differentially expressed genes in children with OSA, with especially a strong association with the DUSP21 gene that was downregulated in the OSA patients.

DUSP21 is a part of the atypical dual specificity phosphatase (DUSP) family, that possess the capability to dephosphorylate Ser/Thr and Tyr residues from proteins as well as to remove phosphates from other non-proteinaceous substrates, including signaling lipids. DUSPs are major modulators of critical signaling pathways and can be deregulated in cancer, obesity, diabetes, inflammation, and Alzheimer's disease.

Little is known about DUSP21 specifically, but it has been previously implicated in hepatocellular carcinoma and colorectal cancer, highlighting its potential role in tumorigenesis through the regulation of Mitogen-Activated Protein Kinase (MAPK) signaling pathways. Here, we show for the first time a possible role for DUSP21 in the pathophysiological mechanisms of OSA in a pediatric population with obesity. It is known that OSA is associated with chronic **IH,** which can lead to systemic inflammation and oxidative stress. Therefore, we hypothesize that *DUSP21* could play a regulatory role in moderating the excessive activation of MAPKs under hypoxic conditions. Even though, DUSP21 was downregulated in patients with OSA, we could not find a dose-response relation between DUSP21 and OSA severity (indicated by oAHI and ODI). It is therefore unclear how DUSP21 is involved in mild versus moderate-to-severe sleep-disordered breathing
The typical DUSP1 gene has also been previously implicated in OSA in both pediatric and adults patients. In the study by Hoffman et al, they showed that intermittent hypoxia is associated with increased DUSP1 expression, which has previously also been proven for sustained hypoxia. Furthermore, the expression of pro-inflammatory cytokines, such as TNF-α, IL1β**,** and IL-1α were enhanced in DUSP1-deficient mice, which aligns with the chronic inflammation seen in OSA. In WO2010051532A1, DUSP1 - amongst other genes - was found to be upregulated in normal weight children with OSA. In our study population, we observed the same upregulated trend of DUSP1.

In conclusion, this study showed that children with obesity and OSA have differentially expressed genes compared to children with obesity alone. Notably, DUSP21 plays a significant role in the pathophysiological mechanisms involved in OSA.

## Claims

1. A method of diagnosing and/or predicting the risk of obstructive sleep apnea (OSA), said method comprising:
a) providing a biological test sample from a subject at risk or suspected having OSA; and
b) determining the expressing of one or more atypical DUSP genes; and
c) wherein the expression of said atypical DUSP genes is compared to a reference value and wherein an alteration in expression in said biological test sample compared to said reference value is indicative for OSA.

2. The method of claim 1, wherein said subject is a subject with overweight or a subject with obesity.

3. The method of claim 1 or 2, wherein said subject is a pediatric subject.

4. The method of any one of claims 1 to 3, wherein said atypical DUSP genes encode for a gene product having a molecular weight of less than 30 kDa, in particular less than 27 kDa, more in particular less than 25 kDa.

5. The method of any one of claims 1 to 4, wherein the expression of at least atypical DUSP21 is determined.

6. The method of claim 5, further comprising the step of determining the expression of one or more genes selected from the list comprising: DUSP3, DUSP11, DUSP12, DUSP13, DUSP15, DUSP18, DUSP19, DUSP22, DUSP23, DUSP24, DUSP27, DUSP28, DUSP14, DUSP26, DUSP1, DUSP2, DUSP4, DUSP5, DUSP6, DUSP7, DUSP8, DUSP9, DUSP10, DUSP16, XIST, MAP7D2, POLR3D, SLC43A3, KLRC3, DAAM2, USP9Y, KDM5D, TTTY15, BCORP1, in particular XIST, POLR3D, MAP7D2, or any combination thereof.

7. The method of any one of claims 1 to 6, wherein said alteration in expression is a decrease in expression of one or more atypical DUSP genes as compared to a reference value and said decrease is indicative for OSA.

8. The method of any one of claims 1 to 7, wherein said reference value is the level of expression of the one or more atypical DUSP genes from a subject who does not have OSA.

9. The method of any one of claims 1 to 8, wherein said expression of one or more atypical DUSP genes is normalized to one or more normalization genes or using count normalization.

10. The method of any one of claims 1 to 9, wherein said expression is determined of RNA transcripts, in particular mRNA transcripts.

11. The method of any one of claims 1 to 10, wherein said biological test sample is selected from the list comprising of: blood, serum, plasma, saliva, or urine.

12. A combination of DUSP21 and one or more genes selected from the list comprising: DUSP3, DUSP11, DUSP12, DUSP13, DUSP15, DUSP18, DUSP19, DUSP22, DUSP23, DUSP24, DUSP27, DUSP28, DUSP14, DUSP26, DUSP1, DUSP2, DUSP4, DUSP5, DUSP6, DUSP7, DUSP8, DUSP9, DUSP10, DUSP16, XIST, MAP7D2, POLR3D, SLC43A3, KLRC3, DAAM2, USP9Y, KDM5D, TTTY15, BCORP1, in particular XIST, POLR3D and/or MAP7D2, for use in the diagnosis and/or prediction of the risk of OSA.

13. A diagnostic kit for performing the method according to any one of the claims 1 to 11, said kit comprising agents for detecting the expression of at least one or more atypical DUSP genes as defined in any one of claims 1 to 12.

14. A method for the reduction and/or treatment of OSA, said method comprising the step of administering to a subject in need thereof an effective amount of a composition comprising one or more atypical DUSP polypeptides or small molecules, or a nucleic acid construct encoding for one or more atypical DUSP polypeptides or small molecules.

15. The method of claim 14, wherein at least one DUSP polypeptide or small molecule is DUSP21.
